(19) Europäisches Patentamt / European Patent Office / Office européen des brevets

(11) **EP 4 316 374 A1**

(12) **EUROPEAN PATENT APPLICATION**

(43) Date of publication:
**07.02.2024 Bulletin 2024/06**

(21) Application number: **22188771.4**

(22) Date of filing: **04.08.2022**

(51) International Patent Classification (IPC):
*A61B 5/243* (2021.01)        *A61B 5/336* (2021.01)

(52) Cooperative Patent Classification (CPC):
**A61B 5/243; A61B 5/336**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Biomagnetik Park Holding GmbH**
**21149 Hamburg (DE)**

(72) Inventor: **PARK, Jai-Wun**
**21149 Hamburg (DE)**

(74) Representative: **Stüven, Ralf**
**RGTH Patentanwälte PartGmbB**
**Kirchenhang 32b**
**21073 Hamburg (DE)**

(54) **MAGNETOGRAPHY FOR THE DETECTION AND CHARACTERIZATION OF INDIVIDUAL CELLULAR ION CURRENTS**

(57) The invention relates to a magnetographic method. The method of the invention allows for the characterization of individual membranous or cytosolic ion currents, for example, ion currents in connection with the action potential of myocardial cells.

**Fig. 2**

**Description**

The invention relates to a magnetographic method and system for the detection and characterization of individual membranous or cytosolic ionic currents.

Non-invasive magnetography is widely used in the field of medicine as a diagnostic tool for diseases of the brain or the heart. Magnetographic apparatus measure biomagnetic fields generated by the electric activity of tissue, e.g., heart or brain tissue. Magnetography is particularly useful in the examination of heart dysfunction, for example, coronary artery disease (CAD).

Magnetography, for example magnetocardiography (MCG), has various advantages over other diagnostic modalities. The recording is contactless and is therefore not influenced by the conductivity of the body. Most importantly, magnetography does not require any form of radiation or contrast media.

CAD is an inflammatory process that initially leads to non-obstructive and eventually obstructive atherosclerotic plaques. The process is exacerbated by various genetic and environmental cardiovascular risk factors [2]. A life-threatening circulatory disturbance (ischemia) occurs when these atherosclerotic plaques of the coronary arteries become severely obstructive. CAD may be accompanied by symptoms such as dyspnoea and chest pain. Both obstructive and non-obstructive stenoses can lead to acute circulatory disturbances of the heart. This can lead to acute plaque rupture, thrombosis and myocardial infarction.

CAD and other causes of myocardial ischaemia still remain the most common cause of death in industrialized countries. Although chest pain is a common symptom in patients with myocardial ischemia, only about 4% of patients who present to a doctor with chest pain actually have an acute coronary syndrome (ACS). Nearly 850,000 patients underwent diagnostic cardiac catheterization in Germany in 2019, but only about 50% required interventional or cardiac surgery consequences (32nd German Heart Report 2020).

In patients with known CAD or a high pre-test probability of CAD, invasive coronary angiography (CTA) is performed. CTA is recommended for PTP between 15% and 50% and allows anatomically accurate morphological visualization of the coronary arteries. The sensitivity is 95-99% which is significantly better than its specificity (64-83%). Some disadvantages are possible contrast media allergy (iodine), contrast media-induced nephropathy/hyperthyroidism, and radiation exposure. Electrocardiographic exercise stress testing followed by potential non-invasive imaging (coronary computed tomography angiography, CTA) is warranted in patients with clinical symptoms and suspected cardiac cause [4].

Whether a noninvasive diagnostic test like electrocardiography (ECG) is appropriate in case of CAD is determined by the CAD pre-test probability. Statistically, these tests are only meaningful in patients with values between 15% and 85%. Clinical scores (for example SYNTAX Score) including age, sex, cardiovascular risk factors, electrocardiogram (ECG) changes, or elevated heart-specific enzymes (troponin) can be used to determine statistically whether patients have a low, intermediate, or high pre-test probability (PTP) of CAD [3]. Individually, chest pain, angina-like symptoms, shortness of breath on exertion, and left arm pain are indicative of potential acute myocardial infarction. In patients with suspected CAD further functional or anatomical testing is indicated. Current international guidelines recommend that the choice of test should be based on the individual's pre-test probability (PTP) of disease. Based on locally available technologies and physician's experience, this may include a coronary CT scan, nuclear imaging test, cardiac MRI, exercise ECG or stress echocardiography.

Exercise ECG has been a standard clinical tool because of its wide availability, low financial expenses and the lack of exposure to radiation, and therefore is still the standard examination procedure in community practices and hospitals, and the one of the basic functional tests in suspected CAD [5]. An ECG stress test for CAD is considered positive if there is at least 1 mm of horizontal or downward ST-segment depression. In addition, ST-segment elevation greater than 1 mm is a strong indication of significant ischemia. However, a meta-analysis of 24,074 patients in 147 studies found that the ECG stress test for the detection of CAD had a sensitivity of only 68% and a specificity of 77% [6]. The sensitivity for the detection of stenosing CAD is only 45-50%. Further, there is generally lower diagnostic reliability in women and limited diagnostic significance in patients undergoing digitalis, nitrate, or β-blocker therapy. In addition, in case of an ST-segment depression at rest of 0-1 mm, only with a pre-test probability $\leq 30\%$ can a negative finding exclude CAD with sufficient probability (< 15%). Summarizing, despite its good availability, low financial expenses and lack of exposure to radiation, there are some serious disadvantages of (exercise) ECG.

Therefore, the 2019 German national guideline on chronic CAD classifies stress ECG only as a second-line diagnostic method, when imaging modalities are not available locally and the pre-test probability is between 15% and 30%. In contrast, diagnostic imaging such as CT angiography (CTA), stress echocardiography, myocardial perfusion SPECT, or stress MRI, are now recommended by national as well as international guidelines when chronic stable CAD is suspected, mainly because these methods not only have a higher diagnostic sensitivity, but are also feasible in patients not being able to undergo an exercise ECG.

It is an object of the present invention to provide an improved, non-invasive method with the aid of which the use of invasive and/or radiation-associated diagnostic methods, in particular in the case of heart diseases, can be reduced or even avoided.

In a first aspect the invention provides a magnetographic method, the method comprising the following steps:

a) Measuring, at least at one given point in time or over at least one given time period, at least one component of a biomagnetic field at least at one position above a tissue or organ of a subject using at least one magnetic field sensor, and

b) Determining, using the magnetic field data measured by the at least one magnetic field sensor, an individual membranous or cytosolic ionic current in the tissue or organ, wherein the determination step is computer-implemented.

The inventors have surprisingly found that magnetography (MG), for example magnetocardiography (MCG), can be used for the detection and/or characterization of cellular, i.e., membranous or cytosolic ionic currents in a tissue or organ of a living subject. The method according to the first aspect of the invention is able to provide previously unavailable data that can be included in a diagnostic parameter or diagnostic parameters for aiding in diagnosing a tissue or organ disorder, for example a heart muscle tissue disorder, in particular for diagnosing the presence or absence of coronary artery disease (CAD).

Based on these findings, it is, for example, possible to detect false positive exercise ECGs based on individual membranous or cytosolic ionic currents. The method of the invention, which will also be called "Cellular Ionography" herein, can thus, for example, advantageously be used in connection with electrography, e.g., electrocardiography, and in particular with exercise ECG. The latter is nearly ubiquitous, and MG is a noninvasive, radiation-free tool. The use of invasive diagnostic methods and/or radiation-associated imaging techniques can thus be reduced or at least be limited to the necessary cases. Although Cellular Ionography will mainly be described here in the context of magnetocardiography (MCG), it is to be noted that it is not limited to the application in relation to myocardial cells or heart tissue.

The term "Cellular Ionography" relates to the detection and characterization of individual cellular ionic currents, i.e., ion currents (fluxes) on a cellular level. This includes membranous cellular ion currents, i.e., ion currents passing the cell membrane, e.g., $Na^+$, $Ca^{2+}$ or $K^+$ ion currents flowing into the cell past the cell membrane via ion channels or out of the cell past the cell membrane via ion channels, or ion currents moving along the cell membrane, and cytosolic ionic currents, i.e., intracellular ion currents not passing the cell membrane, e.g., intracellular $Ca^{2+}$ currents (calcium transients, CaT, ICaT or ICCM) across the sarcoplasmic/endoplasmic reticulum membrane. Such cytosolic currents (for which also the term "subcellular ionic current" or "intracellular ionic current" may be used) remain hidden from electrocardiographic measurement methods due to potential shielding by the cell membrane. The term also encompasses, for example, apparent ion currents along the cell membranes or cytosols of a plurality of neighboring cells, generated or triggered by an action potential propagating along the cell membranes of these cells. It should be noted that, unless explicitly stated otherwise or clear from the context, the terms "cellular ion flux" or "cellular ion current", as used herein, encompass membranous and cytosolic ion fluxes. In particular, the term relates to the detection and characterization of individual ionic currents involved in or occurring during the action potential (AP) at a cell membrane of a living cell.

The terms "radially directed ion flux" or "radial ion flux" relate to ion fluxes (currents) into or out of the cell, i.e., across the cell membrane. The term encompasses ion fluxes apparently moving radially from cell to cell in a tissue, e.g., the myocardium, for example, in the direction from the epicardium to the endocardium, or vice versa. In relation to the sensor plane of a magnetographic sensor arrangement, e.g., a magnetocardiographic sensor arrangement, of a plurality of sensors, the term "radial" can also mean in the direction of the anteroposterior axis.

The terms "tangentially directed ion flux" or "tangential ion flux" relate to ion fluxes (currents) apparently moving along the membranes or along the cytosols of neighboring cells, for example, from the atrioventricular node (AV node) to the apex of the heart, "apparently" because the propagation of depolarization or repolarization from cell to cell appears as a movement of electric charges from cell to cell. In relation to the sensor plane of a magnetographic sensor arrangement, e.g., a magnetocardiographic sensor arrangement, of a plurality of sensors, the term "tangential" can also mean in a direction perpendicular to the anteroposterior axis.

The term "monopolarity" in relation to a magnetic field generated, for example, by the heart, relates to the degree to which a magnetic field deviates from an essentially dipolar shape, i.e., the degree of a shift from an essentially dipolar shape to a more monopolar shape. In particular, the monopolarity represents a measure for a distribution of positive and negative magnetic field sensor signals deviating from a dipolar pattern. A perfect dipolar shape of a magnetic field would result in an axially symmetric isocontour map plotted from measured data of a plurality of sensors, e.g., axial gradiometers, positioned directly above the magnetic field source and oriented in the z-axis using standard heart coordinates. A shift to a more monopolar shape, i.e., a magnetic field having a higher monopolarity, would result in an axially asymmetric isocontour map, where, for example, the majority of the sensors of a plurality of magnetic field sensors positioned directly above the magnetic field source would measure negative or positive values, and/or where the sum of (absolute) negative values is greater than the sum of the positive values, or vice versa. An example of a magnetic field having an essentially "monopolar" shape would, for example, be a magnetic field where all of a plurality of magnetic field sensors positioned directly above the magnetic field source measure only positive or only negative values. It is to be noted here that the term "essentially monopolar magnetic field" or "monopolar magnetic field" does not denote a magnetic monopole, but a magnetic field where one pole (negative or positive) is distributed over a larger area, e.g., of heart tissue, such that no distinct peak (e.g., minus or plus peak) can be determined. The monopolarity can, for example, be determined by taking magnetic field data measured by magnetic field sensors at a specific point in time or over a specific time interval, and comparing, for example, the number of sensors with negative and positive data and/or comparing the magnitudes of the negative or positive data. An inherent offset of the sensors is, of course, taken into account. A perfectly dipolar magnetic field has no (or zero) monopolarity, whereas a magnetic field for which, for example, a larger number of sensors out of a plurality of sensors measuring the magnetic field measures a positive instead of a negative value, or vice versa, has a specific monopolarity (e.g., greater than zero). In particular, the terms "magnetic field having an essentially monopolar shape" or "essentially monopolar magnetic field" relates to a magnetic field where all of a plurality of magnetic field sensors measure either positive or negative values at a given point in time or over a given time interval. The preferably automatic quantification of the monopolarity of a magnetic field may be implemented in different ways. The terms "monopolarity score" or "monopolarity index" are used to refer to a quantified value representing the monopolarity.

The term "dipolarity" in relation to a magnetic field generated, for example, by the heart, relates to the degree to which a magnetic field has a dipolar shape, i.e., a shape with two magnetic field extrema. A perfect dipolar shape would result in an axially symmetric isocontour map plotted from measured data of a plurality of sensors positioned directly above the magnetic field source and oriented in, for example, the z-axis using standard heart coordinates. The terms "dipolarity score" or "dipolarity index" are used to refer to a quantified value representing the dipolarity.

The term "ion flux velocity" relates to the velocity of an ion current in the direction of the ion current, e.g., across the cell membrane into the cytoplasm of the cell. The velocity relates to the number of ions and thus charges per time unit flowing through a unit area of a given cross section, e.g., the cell membrane.

The term "sinus rhythm" relates to the rhythmic sequence of contractions of the heart during the cardiac cycle (heart beat) involving depolarization and repolarization of the atria and ventricles. In the sinus rhythm, depolarisation of the cardiac muscle begins at the sinoatrial node (also called sinus node or SA node) situated in the upper part of the wall of the right atrium, is conducted through the atrioventricular node (AV node) between the atria and ventricles to the bundle of His, the bundle branches (Tawara branches) and the Purkinje fibres. Repolarisation of the ventricles ends the cycle. Depolarisation and repolarisation of the atria and ventricles are reflected by three typical waves or wave complexes on a typical electrocardiogram (ECG), P-wave, QRS complex and T-wave. In the ECG, the P-wave is usually considered to represent atrial depolarization, the QRS complex the depolarization of the right and left ventricles, and the T-wave the repolarization of the ventricles.

The term "J point", $J_p$, relates to the junction between the end of the QRS complex and the beginning of the ST segment of an electrocardiogram.

The term "ST segment" relates to a segment of an electrocardiogram representing the time interval of the cardiac cycle (heartbeat, sinus rhythm) starting after the end of the S-wave of the QRS complex, i.e., at the J point, and ending at the beginning of the T-wave. The ST segment is also called the "ST interval". The ST segment represents the interval between the end of ventricular depolarization and the beginning of ventricular repolarization. The T-wave represents the repolarization of the ventricles. The term "ST-T segment" relates to a segment comprising the ST segment and the T-wave. Although originally created for electrocardiography the terms for describing specific sections of an electrocardiogram (e.g., P wave, QRS complex, ST segment, ST-T segment and T-wave) have been adopted for describing magnetocardiograms, which resemble electrocardiograms in many aspects, although the actual shape may vary depending on the position of the magnetic field sensor.

The term "early repolarization" (ER) relates to a premature repolarization of the action potential resulting in a specific electrocardiographic pattern. According to a current ECG-based definition in Macfarlane et al., 2015, [1], early repolarization is present if all of the following criteria are met: (1) There is an end-QRS notch or slur on the downslope of a prominent R-wave. If there is a notch, it should lie entirely above the baseline. The onset of a slur must also be above the baseline. (2) $J_p$ is ≥0.1 mV in 2 or more contiguous leads of the 12-lead ECG, excluding leads VI to V3. (3) QRS duration is <120 ms.

The term "false positive stress ECG" relates to a stress (exercise) ECG, i.e., electrocardiography performed under defined stress conditions, for example, following the Bruce stress protocol, wherein the electrocardiogram indicates an obstructive coronary artery disease, e.g., through an ST-depression found, but wherein in fact an obstructive coronary artery disease is not present, as, for example, determined by suitable imaging technologies.

The term "systolic electro-magnetic balance point movement", magBPM, relates to a movement of the electromagnetic balance point of the heart during the systole of the cardiac cycle, in particular during the ST-T segment, i.e., between the end of the QRS complex ($QRS_{end}$) or the start of the $I_{to}$ ($I_{to\_beg}$) and the end of the T-wave ($T_{end}$). The electromagnetic balance point of the heart corresponds to the equivalent magnetic dipole source of the magnetic field. The term describes the electrophysiological balance of the heart, and means that the main current, i.e., the maximum current moment, of the heart moves, for example, within an action potential and depends on the movement of the heart and on its electrophysiological cell activity. In normal MCG the balance point is placed in the center of the sensor array in that the position vector of the pseudo-current vector (the directional vector) is placed in the center of the sensor array, and only the rotation of the pseudo-current vector relating to the strongest current moment is monitored. In fact, however, the balance point not only moves with the own movement of the heart during the systole, for example, but also due to physiologic changes, for example, due to a strong radially directed ion flux, e.g., a strong $I_{to}$. The movement of the electromagnetic balance point can, for example, be detected by determining a rotation of the pseudo-current vector relating to the maximum current moment, a shift of the pseudo-current vector relating to the maximum current moment or a rotation of the pseudo-current vector relating to the maximum current moment together with a shift of the pseudo-current vector relating to the maximum current moment in the pseudo-current map. Further, the movement of the electromagnetic balance point can, for example, be determined by vector magnetocardiography, i.e., by determining the trajectory of the pseudo-current vector without placing the position vector of the pseudo-current vector into the center of the sensor array. In a corresponding MCG, the end point of the trajectory of the pseudo-current vector may not fall together with the start point. The direction vectors forming the trajectory would thus not form a closed loop. Depending on the selected signal segment, the distance (in cm or mm) between the start and end point depends on the magBPM, especially during repolarization, e.g., during $QRS_{end}$ or $I_{to\_beg}$ and $T_{end}$. With suitable temporal segmentation of the measured magnetic field data, the magBPM can be determined and even quantified, and can be used, for example, to determine ion flux changes or an untimely ion flux, for example, a premature ion influx or efflux, e.g., an early $I_{to}$, during the end of the QRS complex.

The term "Pole distance" relates to the distance between the extrema of the poles of a dipole in a magnetic field map.

The term "magnetic field map", MFM, has its standard meaning, and refers to a two-dimensional representation of the magnetic field data (usually of the normal (z) component of the magnetic field) measured with magnetic field sensors. The representation can, for example, be in the form of isocontour lines or a color coding for identical magnetic field values (given, e.g., in pT).

The terms "pseudo-current density map", PCDM, "pseudo-current map" or "current field map" have their known meaning and refer to a two-dimensional map representing pseudo-current densities calculated from magnetic field data.

The term "Field map angle" ($\theta_m$) has its standard meaning and relates to the angle of a line joining the negative and the positive poles of a dipole in the magnetic field map, MFM, with a horizontal reference line passing through the center of the MFM.

The term "Maximum current angle" ($\theta_c$) has its standard meaning and relates to the angle between the direction of the pseudo current vector with the highest density (maximum current moment) and a horizontal reference line.

The terms "myocardial disorder" or "myocardial dysfunction" relate to the condition of a non-healthy, i.e., a malfunctioning and/or damaged heart muscle. In particular, the term relates to a malfunctioning and/or damaged heart muscle, e.g., as a result of ischemia.

The term "magnetic field sensor" as used herein means a sensor being able to measure magnetic fields, e.g., a gradiometer. The term comprises a magnetic field sensor (1-axis magnetic field sensor) being designed to only measure one component of the magnetic field, e.g., the z-component, or a magnetic field sensor (2- or 3-axis magnetic field sensor) being able to measure two or all three orthogonal components (x-, y- and z-component) of the magnetic field. SQUIDs ("superconducting quantum interference devices"), e.g., having axial gradiometers as pickup coil, are preferred as sensors.

As used herein, unless not stated otherwise or the context does not indicate otherwise, a reference to an x-axis in relation to the heart or the body of a human being corresponds to a reference to a right-to-left axis, a reference to a y-axis corresponds to a reference to a head-to-foot axis, and a reference to the z-axis corresponds to a reference to an anteroposterior axis. The term "z-component" of a magnetic field thus, for example, refers to the magnetic field component in the direction of the anteroposterior axis, i.e., an axis perpendicular to the x-y plane. The coordinates comprising a x-, y- and z-axis as explained above may also be referred to as "standard heart coordinates".

The term "subject" as used herein refers preferably to a vertebrate, further preferred to a mammal, and most preferred to a human.

The term "measuring a component of the magnetic field at a plurality of positions above a tissue or organ" means measuring a component, e.g., the z-component, of the magnetic field at several positions which are, preferably evenly, spaced apart from each other, e.g., by using an array of magnetic field sensors, for example 36, 52, 64 or 128 magnetic field sensors. The term "above a tissue or organ" is not to be construed as being constricted to a specific position in relation to the earth's gravitation field.

The term "rotational behavior of the measured biomagnetic field" relates to a change of the magnetic field map angle ($\theta_m$) or the maximum current angle ($\theta_c$). The rotation of the biomagnetic field is directly related to a change from a monopolar to a dipolar shape, or vice versa.

The term "determining an individual membranous or cytosolic ionic current in the tissue or organ" relates to the determination of an individual membranous or cytosolic ionic current within or along a cell or a plurality of cells, e.g., multiple cells which are connected in the tissue or organ. The term "multiple cells" includes a plurality of cells which are not interconnected and cells connected with each other within a tissue or organ. The term "ion flux" may also be used for the term "ion current". The term "individual" relates to a specific ion current across a cell membrane or within a cell, e.g., the membranous transient outward potassium current ($I_{to}$) or the cytosolic transient calcium ion current (CaT or ICaT).

In a preferred embodiment of the magnetographic method of the invention, the determination of the individual membranous or cytosolic ionic current in the tissue or organ involves the determination of the magnetic polarity or rotational behavior of the measured biomagnetic field.

Further preferred, in the magnetographic method of the invention the at least one component of the biomagnetic field is measured at a plurality of positions above the tissue or organ of the subject using a plurality of magnetic field sensors, each sensor of the plurality of magnetic field sensors being arranged at one of the positions of the plurality of positions above the tissue or organ of the subject. As an example, the at least one component of the biomagnetic field can be measured at 36, 52, 64, or 128 positions above the tissue or organ of the subject.

In a particular preferred embodiment of the method of the invention, the individual cellular ionic current is an individual cellular ionic current of an action potential (AP) at the cell membrane of a cell, for example a myocardial cell. This encompasses the determination of the individual cellular ionic currents of multiple myocardial cells, e.g., myocardial cells which are connected in a heart tissue. In this embodiment, in particular in an embodiment in which the cell is a myocardial cell, the method of the invention is particularly useful because of its ability to determine, characterize and even quantify the main six partial currents of the action potential of the cell: (i) Sodium current ($I_{Na}$), (ii) L-type calcium current ($I_{CaL}$), (iii) Transient outward potassium current ($I_{to}$), (iv) Rapidly activating delayed rectifying potassium current ($I_{Kr}$), (v) Slowly activating delayed rectifying potassium current ($I_{Ks}$) and (vi) Inward rectifying potassium current ($I_{K1}$). Furthermore, cytosolic currents, particularly the intracellular calcium movement, the so-called calcium transient (CaT, ICCM), which is caused by release of calcium ions from the sarcoplasmic reticulum by ryanodine receptor 2 (RyR2) and their re-uptake into the sarcoplasmic reticulum by the SERCA pump, can be characterized for the first time non-invasively in vivo. This is, for example, useful in the differential diagnosis of Brugada syndrome, especially in its inherited form.

The action potential (AP) of a heartbeat within a cardiac muscle cell is divided into different phases involving different ion fluxes (see, for example, [7]). The cellular ion flow of a heart muscle cell essentially comprises a flow of sodium ($Na^+$) ions, potassium ($K^+$) ions or calcium ($Ca^{2+}$) ions, or a superposition of the mentioned ion flows in any combination. The following currents are involved in the AP of a heart muscle cells, e.g., cells of the Purkinje fibers or the ventricular muscle cells:

$I_{Na}$ = Inward sodium current,
$I_{CaL}$ = L-type calcium current,
$I_{Na/Ca}$ = Sodium calcium exchange (triggers early afterdepolarization (EAD)),
$I_{to}$ = Transient outward potassium current,
$I_{Ks}$ = Slowly activating delayed rectifying potassium current,
$I_{Kr}$ = Rapidly activating delayed rectifying potassium current,
$I_{K1}$ = Inward rectifying potassium current,
$I_{KATP}$ = Adenosine triphosphate-sensitive current,
$I_{KACh}$ = Acetylcholine-activated current.

Inward currents balance with outward currents in the plateau phase of the AP. A disturbance of the intracellular calcium movement ("calcium transient", CaT, ICCM) has an indirect arrhythmogenic effect by triggering EAD (early afterdepolarization = inward current) and DAD (delayed afterdepolarization = inward current).

If the ion flux characteristics are, for example, determined by modeled potential curves, the cellular ion flux of a muscle cell is also in a proportional relationship to the generated magnetic field in a linear or at least linearized modeling of electromagnetism. In electrocardiography, the summed electrical activity of the cardiomyocytes is displayed in an electrocardiogram, the ECG. The ECG signal is generated by released positive charges at the cell membrane, i.e., extra-cellular ion fluxes of the cardiomyocytes. The periodic electrical voltage changes at the body surface or skin are measured. The direct relationship between cardiac contraction and the extra-cellular ionic fluxes can be used to infer the physiological state of the myocardium. The excitation formation system together with the excitation conduction system conditions that the electrical charges successively propagate from cardiomyocyte to cardiomyocyte. That is, the excitation as well as relaxation spreads directionally across the myocardium. It is evident that the electromagnetic field generated by the ion fluxes contains at least equivalent physiological information as the ECG. However, while the electrocardiogram detects electrical potential signals transmitted through matter, the MCG detects the sum of all electromagnetic signals through the corresponding magnetic field signal response. The electromagnetic field is not shielded by matter such as skin and/or a cell membrane and can be measured as well as analyzed by magnetography, e.g., MCG, without loss of information.

It has been found that, in the case of a cardiac muscle cell, for example, MCG parameters can be used for an analysis of (sub-)cellular ion fluxes. This analysis of (sub-)cellular ion fluxes can thus be denoted with the term "Cellular Ionography". With Cellular Ionography, pathologies can be inferred from a generalized human muscle cell physiology, considering medical knowledge, in particular knowledge about ion fluxes. Physiologically expected ion flow characteristics can be compared with preferably digitized and computer-processed measurement signal responses. As an example, early repolarization (ER) can be detected using cellular ionography. MCG parameters proposed herein, e.g., the monopolarity or the rotational behavior of the magnetic field, can advantageously be used to determine whether or not an early repolarization (premature repolarization), e.g., associated with a premature occurrence of $I_{to}$ during the end phase of the QRS complex.

An extension of the analysis to multiple, for example sequentially staggered heartbeats, is also possible. Further, an analysis of the ion fluxes associated with the activity, e.g., the action potential, of the muscle cells during and/or after a physical stress that may be triggered through activity or by medication, is also possible.

In a preferred embodiment of the method of the invention the monopolarity of the magnetic field at at least one given point in time or over at least one given time period is determined. The term "segment" or "time segment" may also be used for the term "time period". The monopolarity of the magnetic field represents a measure for the deviation of the magnetic field from a dipolar shape, and is preferably calculated by the following formula (1)

$$M = \frac{\left|\sum b_i\right|}{\sum \left|b_i\right|} \qquad (1),$$

wherein M is the monopolarity, i the sensor index and $b_i$ the magnetic field gradient measured by the i-th magnetic field sensor.

In this embodiment, the at least one component of the biomagnetic field is preferably measured at a plurality of positions above the tissue or organ of the subject using a plurality of magnetic field sensors. Each sensor of the plurality of magnetic field sensors is arranged at one of the positions of the plurality of positions above the tissue or organ of the subject. The monopolarity is calculated based on the magnetic field data of the plurality of magnetic field sensors. As an example, in case of e.g., 64 magnetic field sensors (i = 1-64) measuring e.g., the z component of the magnetic field, the data measured by each of the 64 sensors is summed up, and the absolute value of this sum is then divided by the sum of the absolute values of each of the sensors. The processing of the magnetic field data of the plurality of magnetic field sensors for calculating the monopolarity is computer-implemented. The magnetic field data are thus digitized and processed by a computer.

The above formula provides a monopolarity score (or monopolarity index) lying between 0 (zero, no monopolarity) and 1 (one, complete monopolarity). A perfect dipolar magnetic field would thus have a zero score, whereas any magnetic field deviating from a dipolar shape and having a more monopolar shape would have a score greater than zero.

The digitized and computer-processed measured magnetic field data can, for example, be visualized two-dimensionally at any sampling point by assigning to each sample a color value from a predetermined color scale. The spatial sensor configuration encodes the arrangement of the respective color values in the two-dimensional image, the so-called magnetic field map, MFP.

A chronological sampling of the underlying magnetic field maps produces a resolution discre-tized in time. In relation to ion fluxes, e.g., during an action potential, a magnetic field map can be analyzed as regards three ion flow characteristics:

(i) Radially directed ion fluxes, such as cellular inward or outward fluxes, produce an electro-magnetic field that results in a monopolar representation in the magnetic field map;
(ii) Tangentially directed ion fluxes, such as ultrafast fluxes along the cell membrane generate an electro-magnetic field that causes a dipolar representation in the magnetic field map;
(iii) Superimposed ion fluxes with different orientations, such as fluxes during a phase transition, can produce a magnetic field map with a multipolar representation.

With the method of the invention, it is thus possible, based on the characteristics mentioned above, to detect and characterize ion fluxes during the phase of (a) repolarization and/or (b) depolarization of an action potential of a myocardial cell or multiple myocardial cells, for example.

As an example, tangential ion fluxes generated during the rapid ventricular depolarization of healthy myocardial cells (phase 0) during the QRS complex result in an essentially dipolar shape of the magnetic field, as visualized in the magnetic field map. The same applies to the slower apparent tangential ion fluxes during ventricular repolarization during the T wave, at least between the start of the T wave ($T_{beg}$) and the peak of the T wave ($T_{max}$), or for the L-type calcium current (ICaL) and the cytosolic calcium transients (CaT). The latter is initiated by the ICaL. Radially directed ion fluxes during initial ventricular repolarization (phase 1), e.g., the $I_{to}$, and the plateau phase (phase 2) result in a more monopolar shape. Any change, in particular any rapid or pronounced change in the polarity of the magnetic field is associated with a change in the underlying ion fluxes. For example, the change from a dominant tangential ion flux during rapid ventricular depolarization during phase 0 of the action potential to the initial repolarization of phase 1 of the action potential that, in healthy subjects, usually occurs at the end of the QRS complex, results in a rapid change from an essentially dipolar shape to a more monopolar shape and thus in an increase of the monopolarity index.

Such an increase can, for example, result in a local maximum of the monopolarity curve, i.e., a curve depicting the monopolarity index over time. If such a local maximum of the monopolarity index can be observed during the QRS complex, for example, during the second half of the downward slope of the QRS complex, this could be an indication of early repolarization due to a rapid radial efflux of $K^+$ ions of $I_{to}$ during the end phase of ventricular depolarization, causing a shift to a more monopolar shape.

In a particular preferred embodiment of the method of the invention, the monopolarity index is at least determined for a time period during the end phase of the QRS complex, particularly preferred during the second half of the downward slope of the QRS complex. For example, the time period during which the monopolarity index is determined may include the last 40 ms, preferably the last 39, 38, 37, 36 or 35 milliseconds of the QRS complex, i.e., before the J point ($J_p$). In this embodiment the method is particularly useful for providing MCG data that can be included in the diagnosis of an early repolarization.

The monopolarity index can be determined for at least one sampling point, for a plurality of discrete, time-separated sampling points or for a time period. The monopolarity index indicates the character of an ion flux, in particular the cellular ion flux trajectories of e.g., $Na^+$, $K^+$, and $Ca^{2+}$ fluxes.

By modifying the monopolarity index using the formula D = 1-M, a dipolarity index D can be obtained. Monopolarity as well as the dipolarity index are normalized superpositions of superimposed cellular ion fluxes and thus are themselves superpositions of cellular ion fluxes. This can be used, for example, to discriminate between radially inwardly or outwardly directed ion fluxes, for example repolarizing $K^+$ or $Na^+$ ion fluxes, from concomitant apparent tangentially directed ion fluxes, for example ion fluxes apparently traveling along the membrane or intracellular $Ca^{2+}$ fluxes propagating from cell to cell during ventricular depolarization. Each monopolarity index and dipolarity index value, can, for example, be normalized by multiplying the index values at a given point in time within a given time period with an average RMS (root mean square) value calculated for the magnetic field data, e.g., those of the z-component of the magnetic field, measured during that time period. The corresponding calculated normalized values for the dipolarity and the monopolarity can be combined in a further calculated value. This allows the splitting of the averaged magnetic field signal into its monopolar and dipolar portions. In this manner, two additional signals are derived from an averaged signal, the first of the signals corresponding to a "monopolar flux", i.e., an ion flux or ion fluxes resulting in a monopolar shape of the magnetic field data in a magnetic field map, and the second signal corresponding to a "dipolar flux", i.e., an ion flux or ion fluxes resulting in a dipolar shape of the magnetic field data in a magnetic field map. Considering the fact that a dipolar flux essentially corresponds to a tangential membranous ion flux or to a tangentially propagating intracellular (cytosolic) ion flux, and a monopolar flux to radially inwardly or outwardly directed ion fluxes, it is possible to relate magnetic field data to activities of cellular ion channels, applying general knowledge about the physiology of a tissue or organ, like the heart, for example.

It is to be noted that a possible inherent sensor offset is, of course, taken into account when determining the monopolarity or dipolarity, i.e., a baseline will be set before computing the monopolarity or dipolarity. It should also be noted that the determination of the monopolarity and/or dipolarity also encompasses a time-resolved determination of monopolarity, M(t), and/or dipolarity, D(t).

Accordingly, the qualitative dynamics of the poles (monopole, dipole, or multipole) within the magnetic field maps, as well as the characteristic course of the aforementioned indices (M, D), individually and/or in combination, decode the subcellular ion flux course associated with the measured magnetic field data. It is thus, for example, possible to determine the kind of ion flux(es) observed at a specific point in time or over a specific time-period and to make, for example, a comparison with physiological ("normal") conditions, e.g., in relation to whether the observed ion flux occurs at the expected time or is early or late.

As an example, the $Na^+$ depolarizing current of phase 0 (rapid influx of $Na^+$ into the cell) of the action potential (AP) is fast and flows in a pronounced rotational motion across the Purkinje fibers, causing it to appear dipolar from the perspective of a magnetocardiography system (MCG), whereas the $I_{to}$ current (transient outward potassium current), which causes early repolarization, does not rotate, but rather flows slowly in a radial, transmyocardial direction, appearing monopolar to the MCG. In comparison, an electrocardiography system (ECG) is, for example, unable to differentiate between the terminal phase of the $Na^+$-depolarization of the right ventricular outflow tract, corresponding to the end phase of the QRS complex in the ECG, and the temporally coincident $I_{to}$ current. Thus, the ECG definition of early repolarization (ER) refers to the amplitude of the J point Jp ≥0.1 mV at the time of phase 1 of the AP and does not consider the actual antecedence of the $I_{to}$ current [1]. It is a regular finding that the early repolarization pattern in ECG disappears with exercise-induced heart rate increase. These findings make it necessary to redefine ER. The so-called false positive stress ECG (absence of epicardial coronary stenosis in coronary angiogram and presence of significant abnormal ST depression in exercise ECG) is caused by an ER with a strong $I_{to}$, which simultaneously causes the ST segment to drop inferiorly due to a pronounced systolic electro-magnetic balance point movement (magBPM, see below) from cranial to caudal. In case that magBPM direction is parallel along the longitudinal heart axis the ECG remains unsuspicious.

Since a rotation of the biomagnetic field is directly related to a change from a monopolar to a dipolar shape, or vice versa, it can be recognized by a strong vector rotation. In a preferred embodiment of the method of the invention, the determination of the rotational behavior of the measured biomagnetic field thus involves the determination of a change of the field map angle and/or the maximum current angle of a pseudo-current vector relating to the maximum current moment, preferably a strong change of the field map angle and/or the maximum current angle. A change is considered strong if it is a change of $\geq 10°$, preferably of $\geq 15°$, $\geq 20°$, $\geq 25°$, $\geq 30°$, $\geq 35°$ or $\geq 40°$, particularly preferred $\geq 45°$, $\geq 50°$, $\geq 55°$ or $\geq 60°$. Such a rotation can, for example, be determined together with a shift of the origin of a pseudo-current vector relating to the maximum current moment.

As mentioned above, the method of the invention can, for example, be particular useful in the diagnosis of early repolarization syndromes, e.g., Brugada syndrome.

The MCG parameters mentioned above, e.g., monopolarity, dipolarity, changes in field map angle and/or the maximum current angle, can be used for a qualitative and quantitative characterization of individual ion currents. Compared with corresponding data of a non-pathological myocardium, this characterization allows for the identification of possible pathological conditions in a subject, e.g., regarding an early repolarization syndrome.

Given an appropriate segmentation in time, the intracellular transient potassium outward ($I_{to}$, ion flow transit outward) measurement signal response can be identified, for example. The $I_{to}$ is a fast radially directed $K^+$ ion flow that initiates repolarization. It causes a dominant monopolelike influence on the magnetic field map. This means that the monopolarity index increases. Therefore, in the case of early repolarization, for example, the monopolarity index forms a local maximum at this point. The $I_{to}$ can be identified with the monopolarity index at this point and quantified by a suitable integration of the monopolarity curve. The magBPM assumes a rotating dynamic in this time segment. Due to the equivalence of magnetic field to the pseudo-current, the monopolar ion influx causes a rotation of the direction vector at least with respect to the pseudo-current vector with maximum current density. Consequently, due to the fast $I_{to}$ inflow ad hoc the structure of the poles within the magnetic field map changes, so that the "Field Map Angle" (the angle describing the orientation of the poles to each other) swings out. Equivalently, a pseudovector rotation can be found in the "Maximum Current Angle" (the angle describing the orientation of the pseudo-current vector with maximum current density).

Within the ST-T segment, immediately after the end of the QRS complex, the depolarizing and repolarizing ion flux dynamics adopt an equilibrium after repolarizing $K^+$ fluxes dominated. The equilibrated ion flux dynamics produce a magnetic field map with a characteristic that is nearly constant in time. In this segment, the monopolarity index assumes a plateau-like course.

From the relation of repolarization with $I_{to}$, the monopolarity index enclosed area, i.e., the area enclosed by the curve representing the course of the monopolarity index over time, can be related to $K^+$ fluxes (inward rectifier). The $Ca^{2+}$ flux is in approximate equilibrium with the inward rectifier and thus can be equated with the area enclosed by the dipolarity index, i.e., the area enclosed by the curve representing the course of the dipolarity index over time. The orientation of the pseudo-current vector with maximum current density remains nearly constant within this phase. The Field Map Angle as well as the Maximum Current Angle take a plateau-like course.

Preferably, in any embodiment of the magnetographic method according to the first aspect of the invention, the at least one component of the biomagnetic field is measured at a plurality of positions above the tissue or organ of the subject using a plurality of magnetic field sensors, each sensor of the plurality of magnetic field sensors being arranged at one of the positions of the plurality of positions above the tissue or organ of the subject.

Especially preferred, the method of the invention is a magnetocardiographic method, wherein the tissue is heart tissue or the organ is the heart of a subject, and wherein the individual cellular ionic current is an individual cellular ionic current of an action potential at the cell membrane of a myocardial cell or multiple myocardial cells. Preferably, the at least one time period includes or consists of the last 40 ms, preferably the last 39, 38, 37, 36 or 35 milliseconds of the QRS complex. Further preferred the individual cellular ionic current preferably is the $I_{to}$.

The measurement of the magnetic field component may be performed under resting conditions, e.g., under conditions where the subject rests on a bed or suchlike, or under stress conditions, i.e., under conditions of physical stress, e.g., during exercises performed with an ergometer or suchlike.

Although it is possible to take any component, i.e., the x-, y- or z-component, of the magnetic field for the determination of its monopolarity, it is preferred to use the z-component for this purpose. The z-component is the component usually measured by magnetocardiography. Using the z-component does not mean that only one-axis magnet field sensors, e.g., gradiometers measuring only z-component, can be implemented. Rather, also 3-axis magnet field sensors measuring the three orthogonal components of the magnetic field can be used, and only the data measured for the z-component are used for the calculation of the monopolarity of the magnetic field.

In the method according to the first aspect of the invention, the step of determining, using the magnetic field data measured by the at least one magnetic field sensor, an individual membranous or cytosolic ionic current in the tissue or organ, e.g., the determination of the monopolarity of the magnetic field, is computer-implemented. The method of the invention is preferably completely automated, in that the magnetic field data measured in step a) are automatically passed in digitized form to step b). The digitized data of step a) are then computed in a suitable manner, e.g., by means of a computer program running, for example, on a personal computer or another programmable apparatus, in order to determine, using the magnetic field data measured by the at least one magnetic field sensor, an individual membranous or cytosolic ionic current in the tissue or organ.

In a second aspect the invention also relates to a magnetographic system, preferably magnetocardiographic system, comprising a plurality of magnetic field sensors being configured to measure a component of the magnetic field generated by a tissue or organ of a subject, and a calculation unit being configured to determine, using the magnetic field data measured by the at least one magnetic field sensor, an individual membranous or cytosolic ionic current in the tissue or organ.

The calculation unit may, for example, be an integrated circuit, a Personal Computer or other programmable apparatus, or part thereof, being configured to perform a calculation. In a preferred embodiment of the second aspect of the invention, the magnetographic system comprises a Personal Computer running a program being configured to determine the monopolarity and/or dipolarity of the magnetic field. The magnetographic system, e.g., magnetocardiographic system, of the invention may thus be composed of a magnetograph being designed and configured to measure, with a plurality of magnetic field sensors, e.g. SQUID sensors coupled to axial gradiometers being configured to measure the z-component of a magnetic field, the magnetic field of a tissue or organ, e.g. the heart of a subject, and a Personal Computer connected to the magnetograph and configured to acquire and process the magnetic field data and to determine, from the magnetic field data, an individual membranous or cytosolic ionic current in the tissue or organ, e.g. by determining the monopolarity and/or dipolarity of the magnetic field, as described in more detail above. A suitable computer program may be used for calculating, for example, a score (index) representing a measure for the monopolarity and/or dipolarity of the magnetic field.

In an especially preferred embodiment of the magnetographic system of the invention the calculation unit is configured to calculate the monopolarity of the magnetic field according to the following formula (1)

$$M = \frac{\left|\sum b_i\right|}{\sum \left|b_i\right|} \quad (1),$$

wherein M is the monopolarity, i the sensor index and $b_i$ the magnetic field gradient measured by the i-th magnetic field sensor, or the dipolarity of the magnetic field by the following formula (2): D = 1-M, D being the dipolarity, and M being the monopolarity, as defined above.

In a further preferred embodiment of the magnetographic system, the system is configured to determine the field map angle and/or the maximum current angle, and to calculate the change of the field map angle and/or the maximum current angle over a given time period, for example, over a time period during the last 40 ms, preferably the last 39, 38, 37, 36 or 35 milliseconds of the QRS complex.

Preferably, the plurality of magnetic field sensors of the magnetocardiographic system of the invention is configured to measure the z-component of the magnetic field. The magnetic field sensors may, for example, be axial first- or second-order gradiometers coupled to a SQUID and oriented in a manner to be able to measure the z-component of the magnetic field, i.e., the component perpendicular to the x-y plane (frontal plane) according to standard heart coordinates. Such sensors are known to the skilled person.

In a further preferred embodiment, the magnetographic method of the invention is a computer implemented magnetocardiographic method for identifying a myocardial disorder associated with early repolarization, ER, the method comprising:

a) measuring magnetic field data for a component of the magnetic field generated by myocardial cells of a heart of a subject measured at a plurality of positions above the heart by a plurality of magnetic field sensors, over at least part of the cardiac cycle comprising at least one time period including at least part of the QRS complex of the sinus rhythm of the heart;
b) determining, using the measured heart magnetic field data, the $I_{to}$ current of the action potential of the myocardial cells.

In a preferred embodiment of this embodiment or the magnetographic method of the invention, the at least one time period preferably includes or consists of the last 40 ms, preferably the last 39, 38, 37, 36 or 35 milliseconds of the QRS complex.

The method allows for the identification and even quantification of a myocardial disorder associated with early repolarization, e.g., Brugada syndrome. For this purpose, the occurrence of the $I_{to}$ current during the action potential of the myocardial cells is determined, and compared with data from normal myocardial cells. A premature occurrence of the $I_{to}$ may be related to a myocardial disorder associated with early repolarization.

In a third aspect the invention relates to a data carrier, i.e., a computer readable medium, comprising a computer program for carrying out the method according to the first aspect of the invention.

In a further aspect the invention relates to the use of magnetography for the detection and characterization of individual membranous or cytosolic ionic currents involved in or occurring during the action potential at the cell membrane of a living cell or multiple cells. Preferably, the living cell is a myocardial cell or the living cells are myocardial cells. In the latter embodiment, the magnetography is magnetocardiography. In a preferred embodiment of the latter embodiment, the magnetocardiography is used for the determination of a false positive stress ECG, as defined above.

In a still further aspect, the invention relates to a magnetocardiographic method, the method comprising the determination of a systolic electro-magnetic balance point movement, magBPM, the systolic electro-magnetic balance point movement being a rotation of a pseudo-current vector relating to the maximum current moment, a shift of the pseudo-current vector relating to the maximum current moment or a rotation together with a shift of the pseudo-current vector relating to the maximum current moment. Alternatively, or additionally, the magBPM is determined by determining the trajectory of the pseudo-current vector with maximum current moment without placing the position vector of the pseudo-current vector with maximum current moment into the center of the sensor array, and determining a distance between the starting point (origin) of the trajectory and the end point of the trajectory. Preferably, the magBPM is determined during the end of the QRS complex or the start of the $I_{to}$, and the end of the T-wave.

In the following the invention is further described for illustration purposes only by way of the attached figures.

Figure 1. Schematic drawing of a simplified magnetocardiogram.

Figure 2. Course of the monopolarity index (here denoted as "PLP score"; top part of the figure) of a part of an average heartbeat of a healthy subject including the QRS complex and the T wave (MCG = dotted line), and a picture of a two-dimensional magnetic field map (bottom left) at time 377 ms.

Figure 3. Course of the monopolarity index ("PLP score"; top part of the figure) of a part of an average heartbeat of a healthy subject including the QRS complex and the T wave (MCG = dotted line), and a picture of a two-dimensional magnetic field map (bottom left) at time 357 ms.

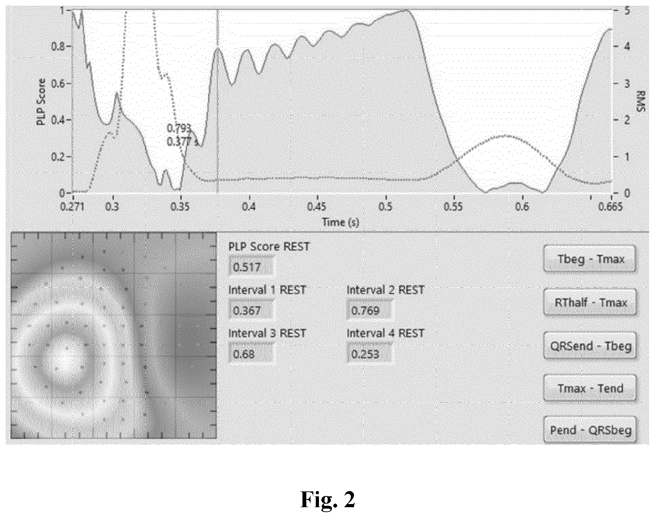

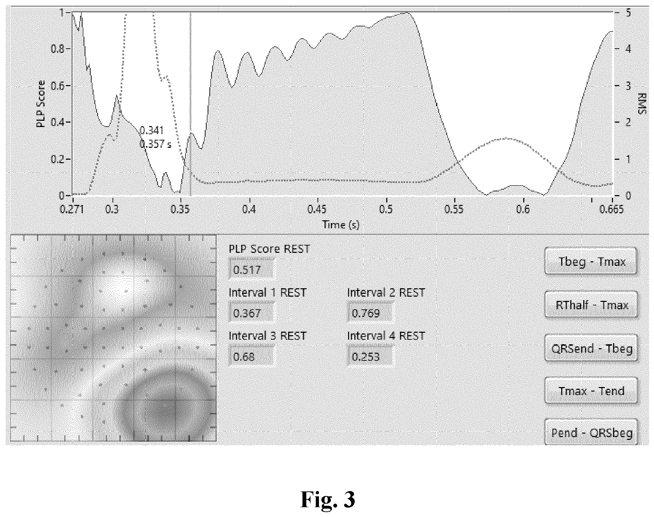

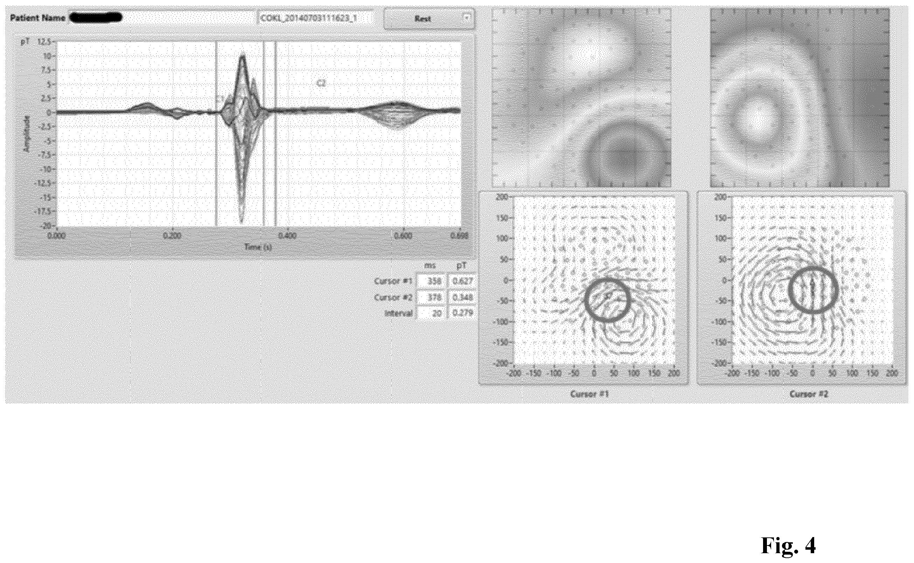

Figure 4. Left: Magnetocardiogram of an average heartbeat of a healthy subject. The magnetic field data of the z-component of a plurality of magnetic sensors are superimposed. Right: Magnetic field maps (top) of figures 2 and 3 at 358 (left, Cursor #1) and 378 ms (right, Cursor #2), and two-dimensional map of pseudo-current vectors. Regarding the pseudo-current vector with the largest current density, a vector rotation is noticeable in the phase transition (see encircled arrows), although the direction of the vector remains almost constant within the repolarization. The change of the location vector, i.e., vector position, indicates a shift of center of gravity due to the activity of the heart.

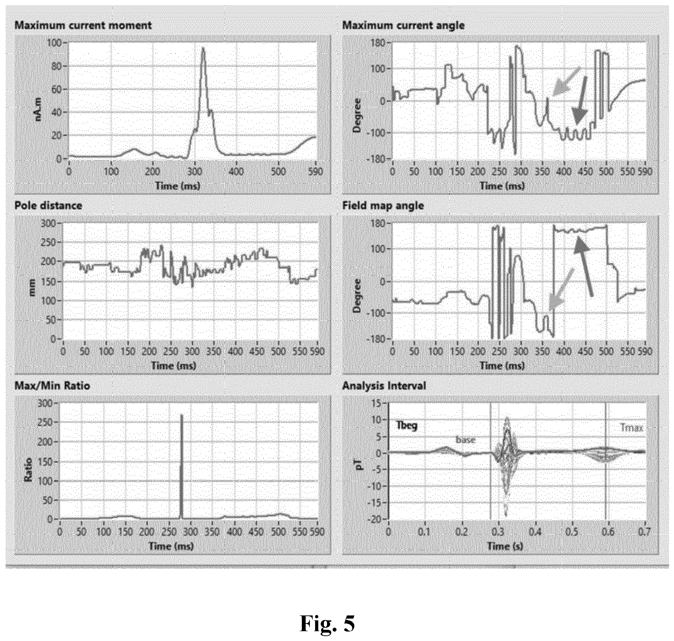

Figure 5. Analysis of the heartbeat shown in figures 2-4. The figure shows the maximum current moment (top, left), the pole distance (middle, left), the Max/Min ration (bottom, left), the maximum current angle (top, right) and the field map angle (middle, right). The bottom right panel shows the full magnetocardiogram (0-700 ms) of figure 4 and the analysis interval (0-590 ms) chosen for the other panels. The light-grey arrow marks the intracellular transient potassium outflow, $I_{to}$, that initiates depolarization; in Maximum current angle (top, right), the peak marked with the light-grey arrow shows vector rotation (see Fig. 4); in Field map angle, the peak marked with the light-grey arrow shows rotation of the dipole; the constant state from the vector field and magnetic field map within repolarization is indicated with the dark-grey arrow.

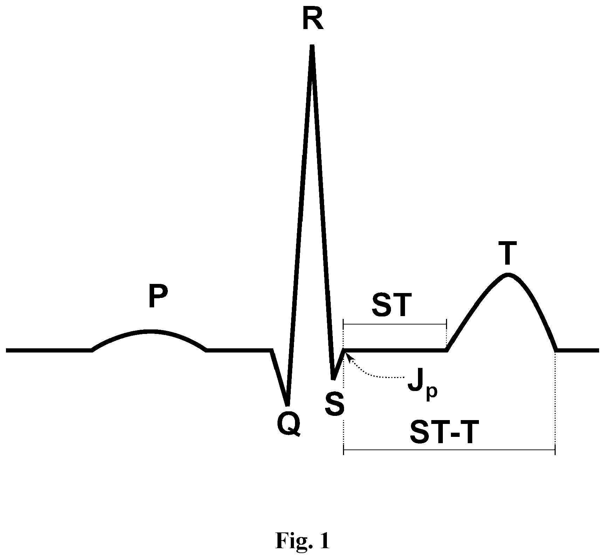

Figure 1 schematically shows an idealized magnetocardiogram of the cardiac cycle of a normal healthy subject. It is to be noted that only the data of one sensor at a specific position is depicted. Magnetocardiograms differ depending on the position of the sensor in relation to the magnetic field. Fig. 1 depicts P-wave, Q-R-S-complex and T-wave, which are well known to the skilled person. The ST segment, the ST-T segment and the J point are also marked.

Figure 2 shows the course of the monopolarity index ("PLP score"; top part of the figure) of a part of an average heartbeat of a healthy subject including the QRS complex, the ST segment and the T wave (MCG = dotted line). In the monopolarity index (light-grey area in the top part of figure 2), the ion fluxes of repolarization are dominant during the ST segment. The magnetic field map (bottom left) increasingly shows a monopolar structure. In Figure 2, the magnetic field map graphically represents the electromagnetic moment at time 377ms (see cursor) within an average heartbeat.

Figure 3 shows the course of the monopolarity index ("PLP score"; top part of the figure) of a heartbeat already shown in Figure 2, but the cursor is now set at time 357 ms, still within depolarization and shortly before repolarization ion fluxes become dominant. The magnetic field map (bottom left) represents magnetic field data at that point in time, showing a dipolar structure. Repolarization is initiated by the $I_{to}$; the electromagnetic moment assumes a local maximum at time 357ms (see cursor), which corresponds to the maximum of the $I_{to}$.

Figure 4 shows a magnetocardiogram of an average heartbeat of a healthy subject (left). The magnetic field data of the z-component of a plurality (here 64) of magnetic sensors are superimposed. In the upper right part of the figure, the magnetic field maps of figures 2 and 3 at 358 (left, Cursor #1) and 378 ms (right, Cursor #2), respectively, are shown. In the lower right part of the figure a two-dimensional map of pseudo-current vectors is shown for the two situations. From the pseudo-current vector maps, using the pseudo-current vector with the largest current density, a vector rotation is noticeable in the phase transition (see encircled arrows), although the direction of the vector remains almost constant within the repolarization. The change of the location vector, i.e., vector position, indicates a shift of center of gravity due to the activity of the heart.

Figure 5 shows an ST-T-Analysis of the heartbeat shown in figures 2-4. The figure shows the maximum current moment (top, left), the pole distance (middle, left), the Max/Min ration (bottom, left), the maximum current angle (top, right) and the field map angle (middle, right). The bottom right panel shows the full magnetocardiogram (0-700 ms) of figure 4 and the analysis interval (0-590 ms) chosen for the other panels. The light-grey arrow marks the intracellular transient potassium outflow, $I_{to}$, that initiates depolarization; in Maximum current angle (top, right), the peak marked with the light-grey arrow shows vector rotation (see Fig. 4); in Field map angle, the peak marked with the light-grey arrow shows rotation of the dipole; the constant state from the vector field and magnetic field map within repolarization is indicated with the dark-grey arrow.

References

[1] Macfarlane PW, et al., "The Early Repolarization Pattern: A Consensus Paper", Journal of the American College of Cardiology 66, No. 4 (28. July 2015): 470-77, https://doi.org/10.1016/j.jacc.2015.05.033.
[2] Erling Falk, Prediman K. Shah, und Valentin Fuster, "Coronary Plaque Disruption", Circulation 92, Nr. 3 (1. August 1995): 657-71, https://doi.org/10.1161/01.CIR.92.3.657.
[3] Ezra A. Amsterdam u. a., "2014 AHA/ACC Guideline for the Management of Patients With Non-ST-Elevation Acute Coronary Syndromes", Journal of the American College of Cardiology 64, Nr. 24 (23. December 2014): e139-228, https://doi.org/10.1016/j.jacc.2014.09.017.
[4] Milad Matta u. a., "Stress Testing and Noninvasive Coronary Imaging: What's the Best Test for My Patient?", Cleveland Clinic Journal of Medicine 88, Nr. 9 (1. September 2021): 502-15, https://doi.org/10.3949/ccjm.88a.20068.
[5] Taylor Dowsley et al., "The Role of Noninvasive Imaging in Coronary Artery Disease Detection, Prognosis, and Clinical Decision Making", Canadian Journal of Cardiology 29, No. 3 (1. March 2013): 285-96, https://doi.org/10.1016/j.cjca.2012.10.022.
[6] Robert Detrano, Renato Gianrossi, und Victor Froelicher, "The Diagnostic Accuracy of the Exercise Electrocardiogram: A Meta-Analysis of 22 Years of Research", Progress in Cardiovascular Diseases 32, Nr. 3 (1. November 1989): 173-206, https://doi.org/10.1016/0033-0620(89)90025-X
[7] Obeyesekere MN, Klein GJ, Nattel S, Leong-Sit P, Gula LJ, Skanes AC, Yee R, Krahn AD A clinical approach to early repolarization. Circulation. 2013 Apr 16;127(15):1620-9. doi: 10.1161/CIRCULATIONAHA.112.143149. PMID: 23588960.

Claims

1. A magnetographic method, the method comprising the following steps:

   a) Measuring, at least at one given point in time or over at least one given time period, at least one component of a biomagnetic field at least at one position above a tissue or organ of a subject using at least one magnetic field sensor, and
   b) Determining, using the magnetic field data measured by the at least one magnetic field sensor, an individual membranous or cytosolic ionic current in the tissue or organ, wherein the determination step is computer-implemented.

2. The magnetographic method of claim 1, wherein the determination of the individual membranous or cytosolic ionic current in the tissue or organ involves the determination of the magnetic polarity or rotational behavior of the measured biomagnetic field.

3. The magnetographic method of claim 2, wherein the monopolarity of the magnetic field at at least one given point in time or over at least one given time period is determined, the monopolarity of the magnetic field representing a measure for the deviation of the magnetic field from a dipolar shape, and wherein the monopolarity of the magnetic field is calculated by the following formula (1)

$$M = \frac{\left|\sum b_i\right|}{\sum |b_i|} \quad (1),$$

wherein M is the monopolarity, $i$ the sensor index and $b_i$ the magnetic field gradient measured by the $i$-th magnetic field sensor.

4. The magnetographic method according to claim 3, wherein a dipolarity D of the magnetic field is calculated with the following formula (2)

$$D = 1 - M$$

D being the dipolarity, and M being the monopolarity.

5. The magnetographic method according to claim 4, wherein the monopolarity M and/or the dipolarity D determined over a period of time are multiplied with an averaged root mean square value calculated for the magnetic field data measured over the period of time.

6. The magnetographic method according to claim 2, wherein the determination of the rotational behavior of the measured biomagnetic field involves the determination of a change of the field map angle and/or the maximum current angle.

7. The magnetographic method according to one of the preceding claims, wherein the at least one component of the biomagnetic field is measured at a plurality of positions above the tissue or organ of the subject using a plurality of magnetic field sensors, each sensor of the plurality of magnetic field sensors being arranged at one of the positions of the plurality of positions above the tissue or organ of the subject.

8. The magnetographic method of one of the preceding claims, wherein the individual membranous or cytosolic ionic current is an individual membranous or cytosolic ionic current of or during an action potential at the cell membrane of a cell or multiple cells.

9. The magnetographic method of one of the preceding claims, wherein the method is a magnetocardiographic method, the tissue being heart tissue or the organ being the heart of a subject, and wherein the individual membranous or cytosolic ionic current is an individual membranous or cytosolic ionic current of or during an action potential at the cell membrane of a myocardial cell or multiple myocardial cells, and wherein the at least one time period preferably includes or consists of the last 40 ms, preferably the last 39, 38, 37, 36 or 35 milliseconds of the QRS complex, and wherein the individual membranous or cytosolic ionic current preferably is the $I_{to}$.

10. The magnetographic method according to one of the preceding claims, wherein the component of the magnetic field is the z-component of the magnetic field.

11. The magnetographic method according to one of the preceding claims, wherein the subject is a mammal, preferably a human.

12. Magnetographic system, comprising a plurality of magnetic field sensors being configured to measure a component of the magnetic field generated by a tissue or organ of a subject, and a calculation unit being configured to determine, using the magnetic field data measured by the at least one magnetic field sensor, an individual membranous or cytosolic ionic current in the tissue or organ.

13. Magnetographic system according to claim 12, wherein the calculation unit is configured to calculate the monopolarity of the magnetic field by the following formula (1)

$$M = \frac{\left|\sum b_i\right|}{\sum \left|b_i\right|} \qquad (1),$$

wherein M is the monopolarity, $i$ the sensor index and $b_i$ the magnetic field gradient measured by the $i$-th magnetic field sensor, or the dipolarity of the magnetic field by the following formula (2): D = 1 - M, D being the dipolarity, and M being the monopolarity.

14. Magnetographic system according to one of claims 12 to 13, wherein the magnetographic system is a magnetocardiographic system.

15. Magnetographic system according to one of claims 12 to 14, wherein the plurality of magnetic field sensors is configured to measure the z-component of the magnetic field.

16. Magnetographic system according to one of claims 12 to 15, comprising a computer being or comprising the calculation unit.

17. Data carrier comprising a computer program for carrying out the method of one of claims 1 to 11.

**Fig. 1**

**Fig. 2**

**Fig. 3**

Fig. 4

**Fig. 5**

Europäisches
Patentamt

European
Patent Office

Office européen
des brevets

# EUROPEAN SEARCH REPORT

Application Number

EP 22 18 8771

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/210127 A1 (KANDORI AKIHIKO [JP] ET AL) 21 October 2004 (2004-10-21) * paragraphs [0003] – [0005], [0009], [0010], [0036], [0042], [0043]; figures 1-5 * ----- | 1-17 | INV. A61B5/243 A61B5/336 |
| X | EP 3 308 703 A1 (BIOMAGNETIK PARK GMBH [DE]) 18 April 2018 (2018-04-18) * paragraphs [0005], [0019] * ----- | 1-17 | |

TECHNICAL FIELDS
SEARCHED       (IPC)

A61B

The present search report has been drawn up for all claims

| Place of search | Date of completion of the search | Examiner |
|---|---|---|
| The Hague | 18 January 2023 | Chau, Thoi Dai |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 ......................................................................
& : member of the same patent family, corresponding
    document

EPO FORM 1503 03.82 (P04C01)

## EP 4 316 374 A1

### ANNEX TO THE EUROPEAN SEARCH REPORT
### ON EUROPEAN PATENT APPLICATION NO.

EP 22 18 8771

This annex lists the patent family members relating to the patent documents cited in the above-mentioned European search report.
The members are as contained in the European Patent Office EDP file on
The European Patent Office is in no way liable for these particulars which are merely given for the purpose of information.

18-01-2023

| Patent document cited in search report | Publication date | Patent family member(s) | | Publication date |
|---|---|---|---|---|
| US 2004210127 A1 | 21-10-2004 | JP | 4426773 B2 | 03-03-2010 |
| | | JP | 2004313608 A | 11-11-2004 |
| | | US | 2004210127 A1 | 21-10-2004 |
| EP 3308703 A1 | 18-04-2018 | EP | 3308703 A1 | 18-04-2018 |
| | | WO | 2018069287 A1 | 19-04-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MACFARLANE PW et al.** The Early Repolarization Pattern: A Consensus Paper. *Journal of the American College of Cardiology,* 28 July 2015, vol. 66 (4), 470-77, https://doi.org/10.1016/j.jacc.2015.05.033
- **ERLING FALK ; PREDIMAN K. SHAH ; VALENTIN FUSTER.** Coronary Plaque Disruption. *Circulation,* 01 August 1995, vol. 92 (3), 657-71, https://doi.org/10.1161/01.CIR.92.3.657
- **EZRA A. AMSTERDAM.** 2014 AHA/ACC Guideline for the Management of Patients With Non-ST-Elevation Acute Coronary Syndromes. *Journal of the American College of Cardiology,* 23 December 2014, vol. 64 (24), e139-228, https://doi.org/10.1016/j.jacc.2014.09.017
- **MILAD MATTA.** Stress Testing and Noninvasive Coronary Imaging: What's the Best Test for My Patient?. *Cleveland Clinic Journal of Medicine,* 01 September 2021, vol. 88 (9), 502-15, https://doi.org/10.3949/ccjm.88a.20068
- **TAYLOR DOWSLEY et al.** The Role of Noninvasive Imaging in Coronary Artery Disease Detection, Prognosis, and Clinical Decision Making. *Canadian Journal of Cardiology,* 01 March 2013, vol. 29 (3), 285-96, https://doi.org/10.1016/j.cjca.2012.10.022
- **ROBERT DETRANO ; RENATO GIANROSSI ; VICTOR FROELICHER.** The Diagnostic Accuracy of the Exercise Electrocardiogram: A Meta-Analysis of 22 Years of Research. *Progress in Cardiovascular Diseases,* 01 November 1989, vol. 32 (3), 173-206, https://doi.org/10.1016/0033-0620(89)90025-X
- **OBEYESEKERE MN ; KLEIN GJ ; NATTEL S ; LEONG-SIT P ; GULA LJ ; SKANES AC ; YEE R ; KRAHN AD.** A clinical approach to early repolarization. *Circulation,* 16 April 2013, vol. 127 (15), 1620-9